**Europäisches Patentamt**

**European Patent Office**

**Office européen des brevets**

(11) Veröffentlichungsnummer: **0 459 371 A1**

# EUROPÄISCHE PATENTANMELDUNG

(21) Anmeldenummer: **91108634.6**

(22) Anmeldetag: **28.05.91**

(51) Int. Cl.5: **C12Q 1/04**

(30) Priorität: **30.05.90 DE 4017398**

(43) Veröffentlichungstag der Anmeldung:
**04.12.91 Patentblatt 91/49**

(84) Benannte Vertragsstaaten:
**AT BE CH DK ES FR GB IT LI LU NL SE**

(71) Anmelder: **Elemér, Faltusz, Dr.**
**Emmelhofer Strasse 7**
**W-7964 Kisslegg(DE)**

(72) Erfinder: **Elemér, Faltusz, Dr.**
**Emmelhofer Strasse 7**
**W-7964 Kisslegg(DE)**

(74) Vertreter: **Riebling, Peter, Dr.-Ing.,**
**Patentanwalt**
**Rennerle 10, Postfach 31 60**
**W-8990 Lindau/B.(DE)**

(54) **Verfahren und Mittel zur Bestimmung der bakteriologischen und zytologischen Beschaffenheit von Rohmilch.**

(57) Die vorliegende Erfindung betrifft ein Verfahren und Mittel zur Bestimmung von mikrobiellen und somatischen Zellen nebeneinander in einer Rohmilchprobe.

Das Verfahren ist dadurch gekennzeichnet, daß man nach Auflösung der störenden Milchbestandteile mittels eines Komplexbildners und eines Tensids, die in der Rohmilchprobe vorliegenden mikrobiellen und somatischen Zellen nach Anfärbung fluoreszenzoptisch auswertet.

EP 0 459 371 A1

Die vorliegende Erfindung ist ein direktes, fluoreszenzopisches Zählverfahren und bietet für die in der Qualitätskontrolle der Rohmilch breit angewandte, automatisierten Routineverfahren, wie z.B. das Bactoscan- und Fossomatic-Verfahren, eine neue Referenzmethode in einem Arbeitsschritt. Darüber hinaus ist das erfindungsgemäße Verfahren auch selbst automatisierbar, wodurch die Qualitätskontrolle betreffend die bakteriologische und zytologische Beschaffenheit der Rohmilch erheblich einfacher, schneller, kostensparender und wegen des relativ niedrigen Chemikalienverbrauchs auch umweltschonender durchführbar wird, als mit den beiden genannten, nebeneinander getrennt arbeitenden Verfahren.

Die Einführung der Kapillarmembranfilter mit definierten Porengrößen in die Mikrofiltrationstechnik eröffnete auch neue Möglichkeiten zur Direktbestimmung von Keimen in Rohmilch.

Im Jahre 1980 wurde das sog. DEFT-Verfahren veröffentlicht. Nach dieser Methode wird die Rohmilchprobe zusammen mit einer Enzymlösung und einer Tensidlösung 10 Min. lang inkubiert, dann durch einen Polycarbonatmembranfilter mit der Porengröße 0,6 $\mu$m filtriert, anschließend mit einer 0,025 %igen Acridinorangelösung überschichtet, erneut filtriert dann zuerst mit einem Citrat-NaOH-Puffer und schließlich mit Ethanol oder Isopropanol gewaschen. Der Membranfilter wird dann getrocknet und die, auf der Oberfläche des Filters befindlichen, gefärbten Keime mit Hilfe eines Fluoreszenzmikroskopes ausgezählt. Die Ergebnisse werden in "Klumpenzahl" und "Einzelkeimzahl" angegegeben.

Wie es später gezeigt wird, sind bei der DEFT-Methode die relativ großen Probenmenge, der 10 Min. lange enzymatische Eiweißabbau, die Anwendung eines hohen Überschuß an Farbstofflösung, sowie die alkoholische Nachbehandlung des Filters teils unnötig, teils komplizierende Details dieses Verfahrens; die hiermit zusammenhängende Problematik dieser Methode ist in der einschlägigen Literatur ausführlich beschrieben.

Als Referenzverfahren für automatisch arbeitenden Routineverfahren, bei denen keine "Klumpenbildung" durch die Aufbereitung der Rohmilchprobe vorliegt, z. B. beim Bactoscan-Verfahren, ist diese Methode ungeeignet.

Scheinbar ganz neue Wege beschreitet eine Patentanmeldung aus dem Jahre 1988. In DE-OS 38 17 089 A1 wird ein Verfahren beschrieben, nach dem ein "spezielles Klärungsreagenz", als Mittel zur Aufbereitung der Rohmilchprobe eingesetzt wird. Dieses Reagenz besteht prinzipiell aus einer Mischung von drei verschiedenen Chemikalien: aus einer anorganischen oder organischen Base, aus einem Alkanol mit zwei bis acht Kohlenstoffatomen, sowie aus einem nichtionischen Tensid. Man gibt diesen Klärungsreagenz zu der Milchprobe bei gegebenen Temperaturen, schüttelt kräftig, worauf sämtliche Milchbestandteile bis auf die Keime und die somatischen Zellen aufgelöst werden. Nach Zugabe einer Fluoreszenzfarbstofflösung wird die Probe entweder indirekt, mit Hilfe eines Durchflußzytometers, oder direkt, mittels einer Neubauer-Zählkammer fluoreszenzoptisch ausgewertet.

Bereits im Jahre 1889 wurde die Röse-Gottlieb-Methode zur gravimetrischen Milchfettbestimmung veröffentlicht. Sie ist heute noch als Referenzverfahren in der Verwendung. Bei dieser Methode wird ebenfalls eine Klärung der Milchprobe durchgeführt, mittels Ammoniak und Ethanol. Es ist daher nicht überraschend, wie dies in der zitierten Patentanmeldung dargestellt wird, die Milch mit der Base und Alkohol zu klären. In der wässrigammoniakalisch-alkoholischen Schicht des Räse-Gottlieb Aufschlusses befindlichen Keime sind nach Anfärbung, z. B. mit einer Acridinorangelösung, ebenfalls mikroskopierbar. Die Anwendung des im DE-OS 38 17 089 A1 aufgeführten Verfahrens als Referenzmethode für die automatisierten Routineverfahren ist denkbar, sofern man für die Direktzählung eine Zählkammer benutzt. Jedoch, wie in der Fachliteratur beschrieben, ist das Auszählen der Bakterienzellen in Zählkammern sehr mühsam, da sie nicht ausnahmslos am Kammerboden sedimentieren. Dagegen sind die Keime bei der Membranfiltertechnik an der Mebranoberfläche fixiert und daher ohne Schwierigkeiten zählbar. Ein Ausweg, die geklärte Probe zuerst durch einen Membranfilter zu filtieren und dann eine Zählung vorzunehmen, scheitert jedoch daran, daß die Kunststoffmembranen in dem streng alkalischen Milieu der Probe zerstört werden.

Nach dem vorliegenden, erfindungsgemäßen Verfahren, wird für die Ermittlung des Keim- und somatischen Zellgehaltes der Rohmilchproben weder eine Enzymlösung, noch die Verwendung von Basen und Alkoholen benötigt. Abgesehen vom Wasser und einem Fluoreszenzfarbstoff, die auch von den oben geschilderten Methoden gleichermaßen verwendet werden, braucht man hier nur noch zwei Reagenzlösungen:
Eine Trimorpholiniumcitrat-Lösung (TMC-Lösung), sowie eine Tensid-Lösung (T-Lösung), beide sind wässrige Lösungen im neutralen pH-Bereich.

Die TMC-Lösung wird erfindungsgemäß im Konzentrationsbereich 0,5 bis 1,0 Mol/Liter verwendet, bevorzugt als 1 Molare Lösung. Man kann anstatt der TMC-Lösung auch andere Komplexbildner, wie z.B. die entsprechenden Salze der 1-Hydroxyäthan-1,1-diphosphonsäure oder der EDTA einsetzen, sie bieten jedoch keine besonderen Vorteile gegenüber dem Citrat.

Als T-Lösung wird erfindungsgemäß eine wässrige Lösung von Decyloxypolyäthylenglycol, mit einem Äthoxylierungsgrad von etwa 11 und mit einem HLB-Wert von etwa 15, im Konzentrationsbereich von 5 bis 25 Gew. %, bevorzugt als eine 20%ige Lösung eingesetzt. Andere, chemisch verwandte, nichtionische Tenside sind ebenfalls verwendbar, sofern sie gleiche oder ähnliche HLB-Werte aufweisen, sie bieten jedoch keine weiteren Vorteile.

Als Farbstoff-Lösung (F-Lösung) wird eine Acridinorange-Lösung bevorzugt verwendet, die man aus einem Konzentrat durch Verdünnung 1:100 mit Wasser für den jeweiligen Tagesbedarf herstellt. Zur Herstellung des Konzentrates werden 0,25 g Acridinorange in einem Liter Wasser gelöst und anschließend durch einen 0,2 μm Membranfilter gefiltert und im Kühlschrank aufbewahrt.

Andere Fluoreszenzfarbstoffe, wie z. B Ethidiumbromid oder Rhodamin B sind ebenfalls anwendbar, ihr bevorzugter Einsatz gegenüber Acridinorange ist jedoch nicht begründet.

Das zur Bereitung der Lösung, sowie als Waschflüssigkeit verwendete Wasser soll bidest Qualität haben, stets frisch destilliert, oder in keim- und partikelfreien Gefäßen abgefüllt und nach Autoklavieren aufbewahrt werden. Sehr gut bewährte sich auch pyrogenfreies Wasser, pro infusione, das im Handel erhältlich ist.

Es ist empfehlenswert, vor Beginn der Messungen einen Blindversuch durchzuführen, um sich zu vergewissern, daß die Lösung und das Wasser frei von Bakterienzellen und anfärbbare Fremdpartikeln sind.

Als weitere Mittel werden noch Kapillarmembranfilter (∅ 25 mm, Porengröße 0,4 μm), eine Filtrationskammer mit eingebautem Magnetrührer, eine geeignete Pinzette, Objektträger, Deckgläser, Immersionsöl, ein Wasserbad, eine Wasserstrahlvakuumpumpe, verschiedene Pipetten, Reagenzgläser mit Schliffstopfen, sowie ein Fluoreszenzmikroskop, ausgerüstet mit den entsprechenden optischen Filtern, benötigt.

Allgemeine Arbeitsvorschrift:

Man temperiert sowohl die Rohmilchprobe, als auch die beiden Reagenzlösungen im Bereich von 40 bis 60 °C, vorzugsweise bei 50 °C. In ein Reagenzglas mit Schliffstopfen wird nun zu einem bestimmten Volumenteil der TMC-Lösung ein Aliquotteil der Rohmilchprobe zugeführt und kräftig geschüttelt, anschließend wird die erfoderliche Menge der T-Lösung hinzupipettiert, man schüttelt erneut kräftig und filtriert dann die optisch klare Mischung durch den Membranfilter durch Anlegen eines nicht zu hohen Vakuums. Nun wird mit Wasser von etwa 20 °C unter Rühren gewaschen. Nach Abstellen des Vakuums wird der Filter mit der F-Lösung überschichtet, kurz weitergerührt und erneut gefiltert. Man holt jetzt den Membranfilter vorsichtig aus der Rührzelle heraus und trocknet ihn bei Zimmertemperatur. Nach dem Trocknen wird der Filter üblicherweise mit Hilfe von Immersionsöl präpariert und die Keime und die somatischen Zellen unter dem Fluoreszenzmikroskop ausgezählt.

Unter Berücksichtigung der Verdünnungsverhältnisse, sowie die erhaltene, durchschnittliche Anzahl der gezählten Keime bzw. Zellen pro Gesichtsfeld, werden bei gegebenen Mikroskopfaktor die Ergebnisse in Anzahl pro ml Milch angegeben.

Die erfoderlichen Mengen der beiden Reagenzlösungen sind in gewissen Grenzen variierbar. Hält man die Milchmenge konstant, so müssen die Mengen der TMC- und der T-Lösung stets so gewählt werden, daß weder die kritische Konzentration der für den Aufschluß erforderlichen Ionenstärke, noch die kritische Mizellenkonzentration (CMC) des jeweiligen Tensides bei gegebener Temperatur unterschritten werden.

Für Reihenuntersuchungen hat sich folgende Arbeitsweise bewährt:

Die Reagenzgläser werden mit gleichen Volumensteilen TMC-Lösung und Rohmilch beschickt und nach Verschließen geschüttelt und ins Wasserbad (50 °C) gestellt. Die Rührzelle wird montiert, mit 2 ml der 50 °C warmen T-Lösung beschickt und der Magnetrührer in Bewegung gesetzt. Mit Hilfe einer Dosierpipette mit Einwegspitze werden zunächst 100 μl aus der TMC-Milch-Mischung hinzupipettiert und gleich anschließend noch 1 ml T-Lösung dazugegeben. Es wird jetzt etwa 10 Sec. lang kräftig, dann 10 Sec. lang sehr langsam weitergerührt, danach wird der Rührer abgestellt und etwa 20 Sec. lang mittels Vakuum (100 bis 200 KPa) gefiltert. Nun wird das Vakuum abgestellt, mit 2ml Wasser (etwa 20 °C) unter Rühren gewaschen und erneut gefiltert. Man überschichtet jetzt den Filter mit 1 ml F-Lösung, rührt sehr langsam etwa 20 Sec. lang, stellt den Rührer ab und filtriert erneut. Man beachte, daß die Ein- und Ausschaltung des Vakuums nie ruckartig geschieht. Die weiteren Schritte werden, wie oben geschildert, ausgeführt.

Ist eine starke Keimbelastung der Rohmilch zu erwarten, so wird die Menge der TMC-Milch-Mischung entsprechend reduziert. Liegen keimarme Milchproben zur Untersuch vor, so kann man den ersten Filtrationsschnitt derart wiederholen, daß man eine zweite 100 μl Portion der TMC-Milch-Mischung auf den gleichen Filter, sonst unter den gleichen Bedingungen, wie oben angegeben, durchfiltriert. Die weiteren Schritte sind in der allgemeinen Arbeitsvorschrift geschildert. Zeitbedarf pro Milchprobe, inklusive Trocknungszeit, die nicht kürzer als 2 Min sein sollte, und Herstellung des Mikroskoppräparates liegt bei einer geübten Person bei etwa 5 Minuten.

Die hier geschilderte einfache und schnelle Methode ist auch geeignet, auotmatisierte Routineverfahren zu überprüfen. Beim Bactoscanverfahren werden zwar die Meßergebnisse in Impulszahlen angegeben, jedoch man kann einen Aliquot aus den Inkubationsbecher des Gerätes membranfiltrieren. Nachdem die Verdünnungsverhältnisse bekannt sind, kann ein Direktvergleich beider Verfahren angestellt werden.

Die Zählung somatischer Zellen in Rohmilch wird nach einer mikroskopischen Direktmethode durchgeführt. Die Methode dient auch als Referenzverfahren zur Herstellung von Standards, sowie zur Standardisierung mechanisierter bzw. automatisierter Zellzählverfahren, wie die elektronische Zählung nach dem Coulter Prinzip, die optische Zählung in der kontinuierlichen Durchflußanalyse (Streulichtmessung), sowie die flureszenzoptische Zählung nach dem Fossomatic-Verfahren.

Bei dem Fossomatic-Verfahren werden 0,2 ml der Milchprobe mit Puffer- und Farblösung gründlich vermischt. Ein Aliquotteil dieser Mischung wird in Form eines dünnen Filmes von 0,5 mm Breite und 10 $\mu$m Dicke auf eine rotierende Scheibe gebracht, die als Objektträger für ein Fluoreszenzmikroskop dient. Jede in ihrem Kern angefärte Zelle erzeugt einen elektrischen Impuls, der nach Verstärkung registriert wird. Die Anzahl der somatischen Zellen wird in 1 000/ml direkt angegeben.

In Rohmilchproben können nach der vorliegenden Erfindung beide Zelltypen nebeneinander bestimmt werden. Da die angefärbten somatischen Zellen von den angefärbten mikrobiellen Zellen in ihrer Größe stark unterscheiden, sind sie mühelos nebeneinander auszählbar.

Das vorliegende Verfahren kann auch automatisiert werden, indem die mit den Reagenzlösungen behandelte Probe nicht membranfiltriert, sondern die Flureszenzintensität der zellulären Partikel mit Hilfe eines Durchflußzytometers direkt in der aufbereiteten Probe gemessen und registriert wird.

Alternativ zu der oben geschilderten Probenaufbereitung kann man vorteilhaft folgenderweise verfahren: Die Milchprobe wird zuerst mit der erforderlichen Menge Morpholin versetzt und anschließend mit einer Tensidlösung, die die stöchiometrische Menge Citronensäure und gegebenenfalls auch das Farbreagenz enthält, kräftig durchgemischt. Durch die Reaktion zwischen Morpholin und Citronensäure entstanden Komplexbildner wird also in der Probe in situ erzeugt und wegen der gleichzeitig auftretenden Reaktionswärme kann auf eine Vortemperierung der Reagenzien verzichtet werden. Dabei können die Milchprobe und die Reagenzien auch durch geeignete Schlauchsysteme zusammengeführt werden.

Beispiel 1:

Zehn Rohmilchproben unterschiedlicher Keimbelastung werden auf 50 °C temperiert und gründlich gemischt. In Reagenzröhrchen werden je 2 ml 1 M Trimorpholiniumcitrat-Lösung von 50 °C mit 2 ml der jeweiligen Rohmilchprobe unter kräftigem Rühren gemischt. Aus dieser Mischung werden Aluquote in Gegenwart von je 3 ml 50 °C warmen Tensid-Lösung nach dem oben geschilderten Verfahren durch Polycarbonatmembranfilter (Porengröße 0,4 $\mu$m) gefiltert, mit je 1 ml Acridinorange-Lösung angefärbt und nach Trocknung die Keime mit dem Flureszenzmikroskop ausgezählt.

Von den gleichen Proben wurde der Gehalt an Bakterien mit dem Bactoscan-Verfahen auch ermittelt, indem man aus dem Gerät Proben entnommen hat, und diese nach Membranfiltration ebenfalls mit dem Flureszenzmikroskop auswertete.

Ein Vergleich der Methoden zeigt die folgende Tabelle:

| Probe | A | B | C |
|---|---|---|---|
| 1 | 510 | $2,6 \times 10^5$ | $3,0 \times 10^5$ |
| 2 | 693 | $3,9 \times 10^5$ | $4,7 \times 10^5$ |
| 3 | 1121 | $6,9 \times 10^5$ | $7,6 \times 10^5$ |
| 4 | 1488 | $1,0 \times 10^6$ | $9,9 \times 10^5$ |
| 5 | 1798 | $1,1 \times 10^6$ | $1,1 \times 10^6$ |
| 6 | 2113 | $1,3 \times 10^6$ | $1,3 \times 10^6$ |
| 7 | 2490 | $1,8 \times 10^6$ | $1,6 \times 10^6$ |
| 8 | 2749 | $2,1 \times 10^6$ | $2,0 \times 10^6$ |
| 9 | 3149 | $2,9 \times 10^6$ | $2,4 \times 10^6$ |
| 10 | 4219 | $3,4 \times 10^6$ | $3,1 \times 10^6$ |

A Bactoscan Impulse (DL 3,0)

B Anzahl der Bakterienzellen/ml; Probe aus dem Bactoscangerät (Inkubationsbecher)

C Anzahl der Bakterienzellen/ml; nach dem erfindungsgemäßen Verfahren

Beispiel 2:

Analog Beispiel 1 werden in drei Rohmilchproben nach der vorliegenden Erfindung die Anzahl der mikrobiellen und der somtischen Zellen bestimmt. Von den gleichen Proben wurden mit dem Bactoscan-Verfahren die Bakterienzellen und mit dem Fossomatic-Verfahren die somatischen Zellen ermittelt. Ein Vergleich der drei Methoden zeigt die folgende Tabelle:

| Probe | A | B | C | D | E |
|---|---|---|---|---|---|
| 1 | 705 | $4,2 \times 10^5$ | $4,6 \times 10^5$ | $2,4 \times 10^5$ | $2,1 \times 10^5$ |
| 2 | 2056 | $9,3 \times 10^5$ | $1,0 \times 10^6$ | $1,4 \times 10^5$ | $1,7 \times 10^5$ |
| 3 | 4950 | $2,9 \times 10^6$ | $3,0 \times 10^6$ | $2,4 \times 10^5$ | $2,6 \times 10^5$ |

A Bactoscan Impulse (DL 3,0)

B Anzahl der Bakterienzellen/ml; Probe aus dem Bactoscangerät
  (Inkubationsbecher)

C Anzahl der Bakterienzellen/ml; nach dem erfindungsgemäßen
  Verfahren

D Anzahl der somatischen Zellen; nach dem Fossomatic-Verfahren

E Anzahl der somatischen Zellen; nach dem erfindungsgemäßen
  Verfahren


Beispiel 3:

Aus einer Rohmilchprobe werden in drei Reagenzröhrchen je 0,5 ml Milch pipettiert. Man gibt dann zu den ersten Röhrchen 3,5 ml 1 M TCM-Lösung und 6,0 ml 20%-ige T-Lösung hinzu, schüttelt kräftig und stellt die Probe für 5 Minuten ins Wasserbad von 50 °C.

35 Teile 1 M TMC-Lösung und 60 Teile 20%-ige T-Lösung werden gemischt. Aus dieser Lösung pipettiert man 9,5 ml zu den zweiten Reagenzröhrchen hinzu, schüttelt kräftig und stellt diese Probe ebenfalls für 5 Minuten ins Wasserbad.

In den dritten Röhrchen wird die Rohmilchprobe zuerst mit 1,35 ml Morpholin versetzt, dann pipettiert man 8,15 ml T-Lösung, die die stöchiometrische Menge Citronensäure enthält, hinzu. Die Probe wird kräftig geschüttelt, worauf sie sich erwärmt. Man läßt sie bei Zimmertemperatur stehen bis sie sich abgekühlt hat. Die erste und die zweite Probe holt man nach etwa 5 Minuten aus dem Wasserbad und kühlt sie ebenfalls auf Zimmertemperatur ab. Man erhält so in den drei Reagenzröhrchen optisch klare Lösungen mit neutralen pH-Werten, die mit Wasser beliebig verdünnbar sind.

Aus den Proben werden gleiche Aliquote bei Zimmertemperatur membranfiltriert, mit etwa 5 ml Wasser gewaschen und anschließend mit 1,0 ml F-Lösung überschichtet. Nach Filtration und Trocknung analog Beispiel 1, werden die drei Proben fluoreszenzoptisch ausgewertet; die erhaltenen Werte zeigen eine sehr gute Übereinstimmung.

## Patentansprüche

1. Verfahren zur Bestimmung von mikrobiellen und somatischen Zellen in Milch, **dadurch gekennzeichnet,**
   daß man nach Auflösung der störenden Milchbestandteile mittels eines Komplexbildners und eines Tensides, die in der Milch vorliegenden mikrobiellen und somatischen Zellen anfärbt und fluoreszenzoptisch nebeneinander auswertet.

2. Mittel zur Durchführung des Verfahrens nach Anspruch 1, enthaltend einen Komplexbildner, ein Tensid und ein Farbreagenz.

3. Mittel nach Anspruch 2, **dadurch gekennzeichnet,** daß der Komplexbildner aus einer wässrigen Trimorpholiniumcitratlösung, das Tensid aus einer wässrigen Decyloxypolyäthylenglycollösung und das Farbreagenz aus einer wässrigen Acridinorangelösung besteht.

4. Mittel nach Anspruch 2, **dadurch gekennzeichnet,** daß der Komplexbildner durch die Reaktion von Morpholin mit Citronensäure, die in der wässrigen Tensidlösung in gelöster Form vorliegt, in der Milchprobe in situ erzeugt wird.

Europäisches
Patentamt

**EUROPÄISCHER
RECHERCHENBERICHT**

Nummer der Anmeldung

**EP 91 10 8634**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 342 501 (MERCK PATENT)<br>* Seite 3, Zeilen 15-18,31-34; Seite 4, Beispiele 1-5 *<br>– – – – – | 1-4 | C 12 Q 1/04 |

| | |
|---|---|
| | RECHERCHIERTE SACHGEBIETE (Int. Cl.5) |
| | C 12 Q |

Der vorliegende Recherchenbericht wurde für alle Patentansprüche erstellt

| Recherchenort | Abschlußdatum der Recherche | Prüfer |
|---|---|---|
| Den Haag | 09 September 91 | VAN BOHEMEN C.G. |